# EUROPEAN PATENT SPECIFICATION

(11) **EP 2 413 894 B1**
(45) Date of publication and mention of the grant of the patent: **04.01.2017**
(21) Application number: 10711977.8
(22) Date of filing: 02.04.2010
(51) Int. Cl.: A61K 8/73

(54) **HAIR-LIKE SHAPED HYDROGELS FOR SOFT TISSUE AUGMENTATION**
HAARARTIGE GEFORMTE HYDROGELE ZUR VERSTÄRKUNG VON WEICHTEILEN
HYDROGELS EN FORME DE CHEVEU POUR AUGMENTATION DES TISSUS MOUS

(30) Priority: 02.04.2009 US 166190 P
(43) Date of publication of application: 08.02.2012
(73) Proprietor: ALLERGAN, INC., Irvine, CA 92612 (US)
(72) Inventor: STROUMPOULIS, Dimitrios, Goleta California 93117 (US); HEREDIA GUILLEN, Karina, Santa Barbara California 93111 (US)
(74) Representative: Hoffmann Eitle
(86) International application number: PCT/US2010/029753
(87) International publication number: WO 2010/115081

(56) References cited:
- EP-A2- 0 466 300
- WO-A1-2008/056069
- WO-A2-2004/092222
- D. W. BUCK ET AL.: "Injectable fillers for facial rejuvenation: a review", JOURNAL OF PLASTIC, RECONSTRUCTIVE ET AESTHETIC SURGERY, vol. 62, 1 January 2009 (2009-01-01), pages 11-18, XP002668828,

## Description

### FIELD OF THE INVENTION

The present invention generally relates to hydrogels useful for soft tissue augmentation, and more specifically relates to a soft tissue filler comprising a hydrogel which is useful for soft tissue augmentation.

### BACKGROUND OF THE INVENTION

Hyaluronic acid (HA), also known as hyaluronan, is a naturally occurring, water soluble polysaccharide, specifically a glycosaminoglycan, which is a major component of the extra-cellular matrix and is widely distributed in animal tissues. HA has excellent biocompatibility and does not cause allergic reactions when implanted into a patient. In addition, HA has the ability to bind large amounts of water, making it an excellent volumizer of soft tissues.

Methods of preparing HA-based soft tissue fillers including both crosslinked and free HA are well known. Crosslinked HA is generally formed by reacting free HA with a crosslinking agent under suitable reaction conditions.

The development of HA-based fillers which exhibit ideal *in vivo* properties as well as ideal surgical usability has proven difficult. For example, HA-based fillers that exhibit desirable stability properties *in vivo,* can be so highly viscous that injection through fine gauge needles is difficult or impossible. Conversely, HA-based fillers that are relatively easily injected through fine gauge needles often have inferior stability properties *in vivo.*

The rate of clearance of an implanted biodegradable material from a location in a body depends on several factors; for example, material shape and size, as well as other mechanisms that can degrade the material into smaller components (e.g. enzymatic or free radical degradation).

Two of the primary clearance mechanisms of implanted biomaterials, for example, implanted HA-based hydrogels used for soft tissue augmentation, are lymphatic drainage and phagocytosis.

Hydrogels intended for soft-tissue augmentation are often formulated to be injectable through a fine gauge needle. This is conventionally accomplished by a process referred to in the industry as "sizing" which generally involves passing a bulk hydrogel material in solid gel form through a sieve multiple times in order to reduce the hydrogel material to micron-sized hydrogel particles which can flow. The hydrogel particles may then be mixed with uncrosslinked HA to improve lubricity in the hydrogel and facilitate its injection through a needle.

WO 2004/092222 discloses a process for crosslinking a mixture of a high molecular weight polysaccharide (e.g. HA) and a low molecular weight polysaccharide.

EP-A-0 466 300 discloses a biocompatible viscoelastic gel slurry comprising swollen, crosslinked gel particles and a polymeric solution.

### SUMMARY OF THE INVENTION

An aspect of the present invention is a method for preparing a soft tissue filler product, the method comprising the steps of:
(i) preparing a crosslinked hydrogel material which comprises crosslinked hyaluronic acid or a crosslinked pharmaceutically-acceptable salt of hyaluronic acid;
(ii) passing the crosslinked hydrogel material through a mesh to form multiple strands of crosslinked hydrogel; and
(iii) packaging the hydrogel product for use as an injectable soft tissue filler while the crosslinked hydrogel is in the form of the multiple strands;
wherein the mesh pore size is 1-200 µm, and each strand of crosslinked hydrogel has a diameter of 1-200 µm and a length which is at least twice the size of the diameter.

This method decreases the extrusion force necessary to extrude crosslinked hydrogels through fine needles and in addition results in a hydrogel with a higher resistance to lymphatic drainage relative to conventionally prepared hydrogels.

Another aspect of the invention is a soft tissue filler comprising a hydrogel product which comprises multiple hydrogel strands, the hydrogel strands comprising crosslinked hyaluronic acid or a crosslinked pharmaceutically-acceptable salt of hyaluronic acid, and the hydrogel strands having a diameter of 1-200 µm and a length which is at least twice the size of the diameter.

The hydrogel strands may have diameters of between 25 µm and 60 µm and lengths of at least 0.1 mm and up to 5 mm.

### BRIEF DESCRIPTION OF THE DRAWINGS

Fig. 1 shows extrusion force test results of a HA-based hydrogel product made in accordance with a method of the present invention and a HA-based hydrogel product made in accordance with prior art methods.
Fig. 2 is a chart showing particle affinity of HA hydrogels made in accordance with methods of the present invention and made in accordance with prior art methods.

### DETAILED DESCRIPTION

In one embodiment, the hydrogel material, prior to being formed into multiple thin strands, comprises a solid mass of crosslinked hyaluronic acid based gel. The solid mass is formed into multiple strands by passing it through a mesh. The mesh has pores of between 1 µm and 200 µm, resulting in strands of material having diameters corresponding to the size of the pores.

In a more particular embodiment, the crosslinked hydrogel material is passed through the mesh a single time prior to being packaged for use as a soft tissue filler product. In other words, the strands of hydrogel are not passed through a mesh a second time, and consequently retain their strand-like or hair-like configuration during subsequent processing steps, and during injection thereof into a target soft tissue site.

Conventional wisdom in the hydrogel art teaches that a mass of crosslinked hydrogel must be reduced down to very small micron-sized particles in order to facilitate extrusion through a fine gauge needle and to encourage a smooth appearance in the skin at the injection site.

It has been a surprising discovery which goes against this conventional wisdom that the substantially non-particulate, hair-like shape of the present hydrogel product is relatively more resistant to lymphatic drainage and phagocytosis, while at the same time requires a relatively low extrusion force for injection through a fine needle. It is theorized by the present inventors that the hair-like shape of the present hydrogels facilitate extrusion thereof through fine gauge needles, possibly, by enabling the hydrogels to align along the direction of flow during injection.

The present invention is also directed toward a soft tissue filler composition having a hair-like or strand like shape, for example, dermal and subdermal fillers, based on hyaluronic acids (HA) and pharmaceutically acceptable salts of HA, for example, sodium hyaluronate (NaHA). As used herein, hyaluronic acid (HA) can refer to any of its hyaluronate salts, and includes, but is not limited to, sodium hyaluronate (NaHA), potassium hyaluronate, magnesium hyaluronate, calcium hyaluronate, and combinations thereof.

Generally, the concentration of HA in the present compositions described herein is preferably at least 10 mg/mL and up to about 40 mg/mL. For example, the concentration of HA in some of the compositions is in a range between about 20 mg/mL and about 30 mg/mL. Further, for example, in some embodiments, the compositions have a HA concentration of about 22 mg/mL, about 24 mg/mL, about 26 mg/mL, or about 28 mg/mL.

The compositions comprise a crosslinked HA-based gel product for injection into soft tissue, wherein the product comprises a HA-composition having a strand-like or hair-like shape. In other words, rather than being spherical or particulate in nature when initially injected into soft tissue, the present hydrogel material comprises multiple thin strands of crosslinked hydrogel material.

The crosslinked hydrogel strands have diameters of between 1 µm and 200 µm and lengths of at least twice, for example, up to 100 times or greater, the magnitude of the corresponding diameter. In some embodiments, the strands have a diameter of between about 25 µm and about 60 µm, and lengths of between about 100 µm up to to several mm, for example up to about 5 mm. The strands may have a generally square, round, angular or other cross-sectional shape, which in some embodiments, depends on the technique for forming the strands from the initial gel. For example, the strands may have cross-sectional shaped substantially conforming to the shape of the pores in a sieve used to form the strands from the initial gel.

Strand length may be somewhat dependent on the cohesivity of the HA composition used to form the strands. Although not intending to be bound by any particular theory of operation, it is hypothesized by the present inventors that gels having relatively high cohesivity will produce longer strands while gels having relatively low cohesivity produce shorter strands. It is believed that gels with lower cohesivity are relatively more brittle and thus break to form smaller strands.

The method comprises preparing a precursor crosslinked HA hydrogel composition, for example, a cohesive, crosslinked HA-based gel, and passing the gel through a mesh to obtain the desired structure. In some embodiments, the gel is passed through a mesh only one time prior to it being used as an injectable product.

In certain embodiments, the precursor composition is a cohesive, hydrated HA-based gel. Such a gel will generally include no greater than between about 1% to about 10% soluble-liquid form or free HA by volume. In certain embodiments, less than about 1% to about 10% of the precursor composition comprises free (i.e. uncrosslinked or lightly crosslinked) HA.

In yet other embodiments, the precursor composition is a relatively non-cohesive, hydrated HA-based gel. Such a "non-cohesive" gel generally includes greater than 10%, greater than about 15%, greater than 20% or more of free HA.

In some embodiments, the precursor composition may comprise a first component made up of relatively highly crosslinked HA in a substantially solid phase, and a second component comprising free or relatively less crosslinked HA in a substantially fluidic phase in which the relatively highly crosslinked HA is dispersed.

In some embodiments, the soft tissue filler composition made from the above-mentioned precursor composition has a somewhat strand-like nature as described elsewhere herein. The composition comprises elongated strands of relatively highly crosslinked HA, dispersed in a medium of free HA.

The strands generally have a substantially uniform diameter and a length that is at least two times, for example, at least three times, at least ten times, at least 20 times, at least 50 times, at least 100 times or greater, the magnitude of the corresponding diameter. In some embodiments, the average diameter of such strands of crosslinked HA is about 1 µm, about 100 µm, or 200 µm.

The precursor composition may be manufactured by pressing a mass of crosslinked HA-based gel through a mesh to create crosslinked HA strands of generally uniform size and shape. These strands may then be mixed with a carrier material, for example, an amount of free HA, to produce a gel product that can be used as an effective soft tissue filler, for example, a facial filler. The gel product is relatively easily extruded through a fine gauge needle in that less force may be required for the extrusion, for example, relative to a substantially identical gel that does not have such a strand-like structure. In some embodiments, the gel product resists degradation, after being placed in the patient, more readily relative to a substantially identical gel that does not have such a strand-like structure.

Manufacturing of the present HA compostions may comprise, in one embodiment, the initial step of providing raw HA material in the form of dry HA fibers or powder. The raw HA material may be HA, its salts and/or mixtures thereof. The HA material may comprise fibers or powder of NaHA, and in some embodiments, bacterial-sourced NaHA. Alternatively, the raw HA material may be animal derived. The HA material may be a combination of raw materials including HA and at least one other polysaccharide, for example, glycosaminoglycan (GAG).

In some embodiments, the HA material in the compositions nearly entirely comprises or consists of high molecular weight HA. That is, nearly 100% of the HA material in the present compositions may be high molecular weight HA as defined below. In other embodiments, the HA material in the compositions comprises a combination of relatively high molecular weight HA and relatively low molecular weight HA, as defined below.

High molecular weight HA as used herein describes a HA material having a molecular weight of at least about 1.0 million Daltons (mw ≥ 10⁶ Da / 1.0 MDa) to about 4.0 MDa. For example, the high molecular weight HA in the present compositions may have a molecular weight of about 2.0 MDa. In another example, the high molecular weight HA may have a molecular weight of about 2.8 MDa.

Low molecular weight HA as used herein describes a HA material having a molecular weight of less than about 1.0 MDa. Low molecular weight HA can have a molecular weight of between about 200,000 Da (0.2 MDa) to less than about 1.0 MDa, for example, between about 300,000 Da (0.3 MDa) to about 750,000 Da. (0.75 MDa).

The HA material of the compositions may comprise between about 5% to about 95% high molecular weight HA with the balance of the HA material including low molecular weight HA. In one embodiment of the invention, the ratio of high molecular weight to low molecular weight HA is at least about, and preferably greater than 2 (w/w ≥ 2) with the high molecular weight HA having a molecular weight of above 1.0 MDa.

It will be appreciated by those of ordinary skill in the art that the selection of high and low molecular weight HA material and their relative percentages or ratios is dependent upon the desired characteristics, for example, extrusion force, elastic modulus, viscous modulus and phase angle expressed as the ratio of viscous modulus to elastic modulus, cohesivity, etc. of the final HA-based product.

The HA-based gels can be prepared according to the present invention by first cleaning and purifying the dry or raw HA material having a desired high/low molecular weight ratio. These steps generally involve hydrating the dry HA fibers or powder in the desired high/low molecular weight ratio, for example, using pure water, and filtering the material to remove large foreign matters and/or other impurities. The filtered, hydrated material is then dried and purified. The high and low molecular weight HA may be cleaned and purified separately, or may be mixed together, for example, in the desired ratio, just prior to crosslinking.

In accordance with the present invention, pure, dry NaHA fibers are hydrated in an aqueous solution, for example, a neutral, slightly acidic or alkaline solution, to produce a free NaHA gel. In one embodiment, a suitable alkaline solution may be used to hydrate the NaHA, for example, aqueous solutions containing sodium hydroxide (NaOH), potassium hydroxide (KOH), sodium bicarbonate (NaHCO₃), lithium hydroxide (LiOH), and the like. In another embodiment, the suitable alkaline solution is an aqueous solution containing NaOH. The resulting alkaline gel will have a pH above 7.5. The pH of the resulting alkaline gel can have a pH greater than 9, or a pH greater than 10, or a pH greater than 12, or a pH greater than 13.

The manufacturing process further involves the step of crosslinking the hydrated NaHA gel with a suitable crosslinking agent. The crosslinking agent may be any agent known to be suitable for crosslinking polysaccharides and their derivatives via their hydroxyl groups. Suitable crosslinking agents include, but are not limited to, 1,4-butanediol diglycidyl ether (or 1,4-bis(2,3-epoxypropoxy)butane or 1,4-bisglycidyloxybutane, all of which are commonly known as BDDE), 1,2-bis(2,3-epoxypropoxy)ethylene and 1-(2,3-epoxypropyl)-2,3-epoxycyclohexane. The use of more than one crosslinking agent or a different crosslinking agent is not excluded from the scope of the present invention. In one embodiment, the HA gels described herein are crosslinked using BDDE.

The step of crosslinking may be carried out using any means known to those of ordinary skill in the art. Those skilled in the art appreciate how to optimize conditions of crosslinking according to the nature of the HA, and how to carry out crosslinking to an optimized degree. Degree of crosslinking for purposes of the present invention is defined as the percent weight ratio of the crosslinking agent to HA-monomeric units within the crosslinked portion of the HA based composition. It is measured by the weight ratio of HA monomers to crosslinker (HA monomers:crosslinker).

In some embodiments, the HA is crosslinked during the step of hydration of the raw HA fibers. In other embodiments the HA is crosslinked after the step of hydration of the raw HA fibers.

The degree of crosslinking in the HA component of the present compositions is at least about 2% and is up to about 20%. In other embodiments, the degree of crosslinking is greater than 5%, for example, is about 6% to about 8%. In some embodiments, the degree of crosslinking is between about 4% to about 12%. In some embodiments, the degree of crosslinking is less than about 6%, for example, is less than about 5%.

In some embodiments, the HA gel is capable of absorbing at least about one time its weight in water. When neutralized and swollen, the crosslinked HA component and water absorbed by the crosslinked HA component is in a weight ratio of about 1:1.

Once the HA gel is made by mixing the desired high and low molecular weight ratios of dry HA fibers, hydrating the dry fibers and crosslinking the HA component to the desired degree, the next step of the present invention involves shaping or forming the strand-like hydrogel. Shaping or forming of the strand-like hydrogel is accomplished by passing the crosslinked HA gel mass through a mesh to cut through the mass of gel and thereby form the strand-like shaped hydrogel therefrom. In accordance with a particular embodiment, the strand-like hydrogel is not subjected to any further cutting, shaping or sizing steps. In one embodiment, the precursor HA gel is passed through a mesh only one time prior to the final product being packaged in a syringe for use as a soft tissue filler. It is contemplated that this shaping or forming step may, in some instances, be repeated in accordance with other embodiments of the invention, so long as the resulting hydrogels retain their strand-like shape.

### EXAMPLE 1

### Preparation of a HA soft tissue filler product according to the present invention

1 gram of sodium hyaluronate fibers (NaHA, Mw = 0.5-3 MDa) is mixed with 5-10 g of 1% sodium hydroxide solution and the mixture is allowed to hydrate for 1-5 hrs forming a hydrated NaHA gel. 50-200 mg of 1,4-butanediol diglycidyl ether (BDDE) are added to the NaHA gel and the mixture is mechanically homogenized.

The mixture is then placed in a 40-70°C oven for 1-4 hrs. The resulting crosslinked hydrogel is neutralized with an equimolar amount of hydrochloric acid (HCl) and swelled in phosphate buffered saline, (PBS, pH 7). The hydrogel is sized by passing it through a 25 µm or 43 µm mesh screen one (1) time. After being passed through the mesh screen a single time, the resulting thin, hair-like strands of hydrogel are dialyzed, packaged and sterilized.

### EXAMPLE 2

### Preparation of a HA Filling Gel by the Process of the PRIOR ART

1 gram of sodium hyaluronate fibers (NaHA, Mw = 0.5-3 MDa) is mixed with 5-10 g of 1% sodium hydroxide solution and the mixture is allowed to hydrate for 1-5 hrs. 50-200 mg of 1,4-butanediol diglycidyl ether (BDDE) are added to the NaHA gel and the mixture is mechanically homogenized.

The mixture is then placed in a 40-70°C oven for 1-4 hrs. The resulting crosslinked hydrogel is neutralized with an equimolar amount of hydrochloric acid (HCl) and swelled in PBS (pH 7). The hydrogel is sized by passing it through a 105 µm mesh screen seven (7) times. After being passed through the mesh screen seven times, the resulting micron-sized hydrogel particles are dialyzed, packaged and sterilized.

### COMPARISON 1

### Continuous Extrusion Force Test

To evaluate the rheological properties of the HA filling gels prepared in Examples 1 and 2, continuous extrusion force tests were performed. This test measures the force needed to pass the gel through a needle. Specifically, the lower the extrusion force, the easier it is to extrude a gel. Extrusion forces less than 40 N through a 30G needle are desirable for injection into soft tissue.

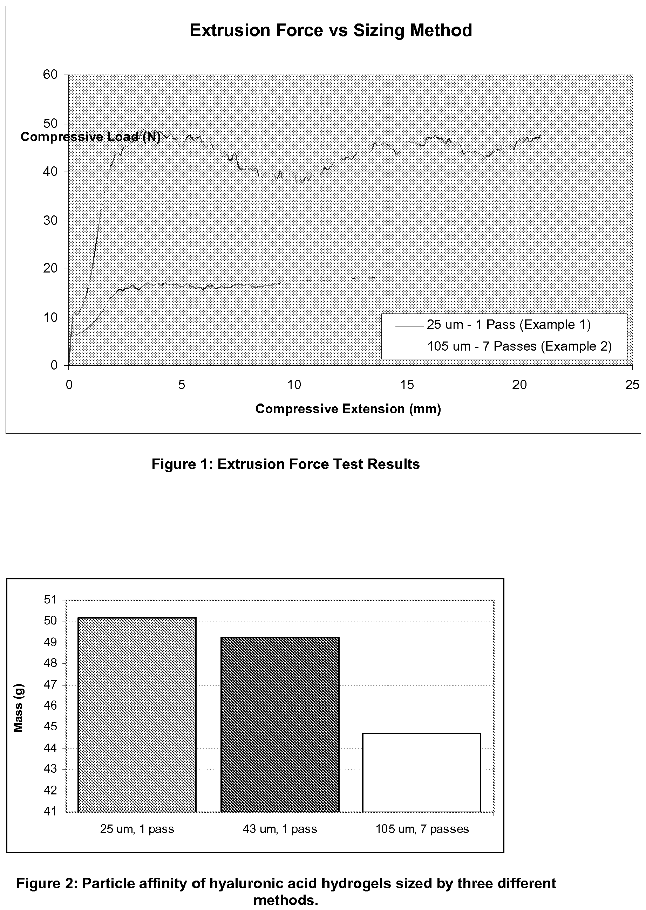

The extrusion force tests were performed on an Instron instrument using a 1 mL syringe with a 27G needle. 0.5 mL of each sample was extruded at a constant rate of 50 mm/min. The peak force recorded quantifies the ease of extrusion. The compressive force as a function of the compressive extension for the two samples is plotted in Figure 1. The results show that the extrusion force peak recorded for the gel prepared by the process of the invention is significantly lower than that recorded for the process of the prior art. Further, the extrusion force profile for the former case is smoother as demonstrated by a relatively flat plateau.

### COMPARISON 2

### Particle Affinity

To assess the cohesivity of the gels, particle affinity measurements were performed. This assay indirectly measures the affinity the gel has for itself by measuring the mass of 5 gel droplets formed while extruding through a 30 gauge needle at a constant rate. A gel with a higher particle affinity (i.e. more cohesive/sticky) will have larger and heavier droplets. Three gels were synthesized as described above, and sized by three different methods. The first method was via 1 pass through a 25 µm mesh and the second was passed 1 time through a 43 µm mesh, forming hair-like gel. The third sizing method was performed by passing the gel 7 times through a 105 µm mesh. This results in a particulate gel. Shown in Figure 2 are the particle affinity results. The gels passed 1 time through the 25 and 43 µm mesh, have higher particle affinities than the particulate gel formed from multiple passes through the 105 µm mesh.

### EXAMPLE 3

NaHA fibers or powder are hydrated in an alkaline solution, for example, an aqueous solution containing NaOH. The mixture is mixed at ambient temperature, about 23°C, to form a substantially homogenous, alkaline HA gel.

A crosslinking agent, BDDE, is diluted in an aqueous solution and added to the alkaline HA gel. The mixture is homogenized for several minutes.

Alternatively, BDDE can be added directly to the HA fibers (dry state) at the beginning of the process, prior to the hydration. The crosslinking reaction will then start relatively slowly at ambient temperature, ensuring even better homogeneity and efficacy of the crosslinking. Methods of crosslinking polymers in the dry state using a polyfunctional crosslinking agent such as BDDE are described in, for example, Piron et al., U.S. Patent No. 6,921,819 which is incorporated herein by reference in its entirety as if it were part of the present specification.

The resulting crosslinked HA gel mixture is then heated at about 50°C for about 2.5 hours. The material is now a highly crosslinked HA/BDDE gel (aspect = solid gel). This crosslinked gel is then neutralized with a suitable acidic solution. The neutralized HA gel is then swollen in a phosphate buffer at a cold temperature, for example a temperature of about 5°C, to obtain a highly cohesive HA gel. In this specific example, the phosphate buffered saline solution contains water-for-injection (WFI), disodium hydrogen phosphate, and sodium dihydrogen phosphate. When neutralized and swollen, the crosslinked HA component and water absorbed by the crosslinked HA component is in a weight ratio of about 1:1. The hydrogel is then passed through a mesh screen one (1) time (screen pore diameter 25µm - 60µm) creating a HA gel comprising hair-like strands having diameters about equivalent to the screen pore diameter and lengths generally between about 0.5 mm and about 3mm.

The hair-like HA gel is then mechanically stirred and filled into dialysis membranes and dialyzed against a phosphate buffer. The gel is then filled into dialysis membranes and dialyzed against a phosphate buffer for up to several days with regular changes of the bath, in order to remove the un-reacted crosslinker, to stabilize the pH close to neutrality (pH=7.2) and to ensure proper osmolarity of the HA gel. The gel is then packaged into syringes for dermal injection and sterilized in accordance with conventional means.

### EXAMPLE 4

The hair-like shaped HA gel is made in accordance with the method described in EXAMPLE 3, except that prior to packaging into syringes, lidocaine chlorhydrate (lidocaine HCl) is added. First, lidocaine HCl in powder form is first solubilized in WFI and filtered through a 0.2 µm filter. Dilute NaOH solution is added to the HA gel in order to reach a slightly basic pH (for example, a pH of between about 7.5 and about 8). The lidocaine HCl solution is then added to the slightly basic gel to reach a final desired concentration, for example, a concentration of about 0.3% (w/w). The resulting pH of the HA/lidocaine mixture is then about 7 and the HA concentration is about 24 mg/mL. Mechanical mixing may be performed in order to obtain a proper homogeneity.

If desired, a suitable amount of free HA gel may be added to the HA/lidocaine gel mixture with the advantage of increasing the kinetics of lidocaine delivery. For example, free HA fibers are swollen in a phosphate buffer solution, in order to obtain a homogeneous viscoelastic gel. This free HA gel is then added to the crosslinked HA/lidocaine gel (for example, at about 5%, w/w). The resulting gel is then filled into sterile syringes and autoclaved at sufficient temperatures and pressures for sterilization for at least about 1 minute.

After autoclaving, the final HA/lidocaine product is packaged and distributed to physicians. The product manufactured in accordance with this method exhibits one or more characteristics of stability as defined elsewhere herein. For example, the autoclaved HA/lidocaine product has a viscosity, cohesivity, and extrusion force that are acceptable. No degradation of the HA/lidocaine gel product is found during testing of the product after the product has spent several months in storage.

## Claims

1. A method for preparing a soft tissue filler product, the method comprising the steps of:
(i) preparing a crosslinked hydrogel material which comprises crosslinked hyaluronic acid or a crosslinked pharmaceutically-acceptable salt of hyaluronic acid;
(ii) passing the crosslinked hydrogel material through a mesh to form multiple strands of crosslinked hydrogel; and
(iii) packaging the hydrogel product for use as an injectable soft tissue filler while the crosslinked hydrogel is in the form of the multiple strands;
wherein the mesh pore size is 1-200 µm, and each strand of crosslinked hydrogel has a diameter of 1-200 µm and a length which is at least twice the size of the diameter.

2. A method according to Claim 1, wherein the crosslinked hydrogel material is passed through the mesh a single time prior to being packaged for use as a soft tissue filler product.

3. A method according to Claim 1 or Claim 2, wherein the step of preparing a crosslinked hydrogel material comprises combining hyaluronic acid or a pharmaceutically-acceptable salt thereof with at least one crosslinking agent selected from 1,4-butanediol diglycidyl ether (BDDE), 1,4-bis(2,3-epoxypropoxy)butane, 1,4-bisglycidyloxybutane, 1,2-bis(2,3-epoxypropoxy)ethylene, 1-(2,3-epoxypropyl)-2,3-epoxycyclohexane and divinyl sulfone (DVS).

4. A method according to any preceding claim, wherein the crosslinked hydrogel material comprises crosslinked sodium hyaluronate.

5. A method according to Claim 4, wherein the sodium hyaluronate is crosslinked with BDDE.

6. A method according to Claim 1, further comprising the step of adding a lubricant to the multiple strands prior to the step of packaging.

7. A method according to Claim 6, wherein the lubricant is an uncrosslinked hydrogel.

8. A soft tissue filler comprising a hydrogel product which comprises multiple hydrogel strands, the hydrogel strands comprising crosslinked hyaluronic acid or a crosslinked pharmaceutically-acceptable salt of hyaluronic acid, and the hydrogel strands having a diameter of 1-200 µm and a length which is at least twice the size of the diameter.

9. A filler according to Claim 8, wherein each hydrogel strand has a diameter of 1-60 µm.

10. A filler according to Claim 9, wherein each hydrogel strand has a diameter of 25-60 µm.

11. A filler according to Claim 8, wherein each hydrogel strand has a length of at least 0.1 mm.

12. A filler according to Claim 11, wherein each hydrogel strand has a length of 0.1-5 mm.

13. A filler according to Claim 12, wherein each hydrogel strand has a length of 0.5-3 mm.

14. A filler according to Claim 8, wherein each hydrogel strand has a diameter of 25-60 µm and a length of 0.1-5 mm.

## Patentansprüche

1. Verfahren zur Herstellung eines Weichgewebe-Füllstoffprodukts, wobei das Verfahren die Schritte umfasst:
(i) Herstellen eines vernetzten Hydrogelmaterials, das vernetzte Hyaluronsäure oder ein vernetztes, pharmazeutisch annehmbares Salz von Hyaluronsäure umfasst;
(ii) Passieren des vernetzten Hydrogelmaterials durch ein Sieb, um mehrere Stränge von vernetztem Hydrogel zu bilden; und
(iii) Verpacken des Hydrogelprodukts zur Verwendung als injizierbarer Weichgewebe-Füllstoff, während das vernetzte Hydrogel in Form von mehreren Strängen vorliegt;
wobei die Siebporengrösse 1 bis 200 *µ*m beträgt, und jeder Strang aus vernetztem Hydrogel einen Durchmesser von 1 bis 200 *µ*m und eine Länge, die zumindest das Zweifache des Durchmessers beträgt, aufweist.

2. Verfahren gemäss Anspruch 1, wobei das vernetzte Hydrogelmaterial ein einziges Mal durch das Sieb passiert wird, bevor es zur Verwendung als Weichgewebe-Füllstoffprodukt verpackt wird.

3. Verfahren gemäss Anspruch 1 oder Anspruch 2, wobei der Schritt der Herstellung eines vernetzten Hydrogelmaterials das Kombinieren der Hyaluronsäure oder eines pharmazeutisch annehmbaren Salzes davon mit zumindest einem Vernetzungsmittel, ausgewählt aus 1,4-Butandioldiglycidylether (BDDE), 1,4-Bis(2,3-epoxypropoxy)butan, 1,4-Bisglycidyloxybutan, 1,2-Bis(2,3-epoxypropoxy)ethylen, 1-(2,3-Epoxypropyl)-2,3-epoxycyclohexan und Divinylsulfon (DVS), umfasst.

4. Verfahren gemäss irgendeinem der vorhergehenden Ansprüche, wobei das vernetzte Hydrogelmaterial vernetztes Natriumhyaluronat umfasst.

5. Verfahren gemäss Anspruch 4, wobei das Natriumhyaluronat mit BDDE vernetzt ist.

6. Verfahren gemäss Anspruch 1, das ferner einen Schritt umfasst, in dem ein Gleitmittel vor dem Verpackungsschritt zu den mehreren Strängen zugegeben wird.

7. Verfahren gemäss Anspruch 6, wobei das Gleitmittel ein unvernetztes Hydrogel ist.

8. Weichgewebe-Füllstoff, umfassend ein Hydrogelprodukt, das mehrere Hydrogelstränge umfasst, wobei die Hydrogelstränge vernetzte Hyaluronsäure oder ein vernetztes, pharmazeutisch annehmbares Salz von Hyaluronsäure umfassen und die Hydrogelstränge einen Durchmesser von 1 bis 200 *µ*m und eine Länge, die zumindest das Zweifache des Durchmessers beträgt, aufweisen.

9. Füllstoff gemäss Anspruch 8, wobei jeder Hydrogelstrang einen Durchmesser von 1 bis 60 *µ*m aufweist.

10. Füllstoff gemäss Anspruch 9, wobei jeder Hydrogelstrang einen Durchmesser von 25 bis 60 *µ*m aufweist.

11. Füllstoff gemäss Anspruch 8, wobei jeder Hydrogelstrang eine Länge von zumindest 0,1 mm aufweist.

12. Füllstoff gemäss Anspruch 11, wobei jeder Hydrogelstrang eine Länge von 0,1 bis 5 mm aufweist.

13. Füllstoff gemäss Anspruch 12, wobei jeder Hydrogelstrang eine Länge von 0,5 bis 3 mm aufweist.

14. Füllstoff gemäss Anspruch 8, wobei jeder Hydrogelstrang einen Durchmesser von 25 bis 60 *µ*m und eine Länge von 0,1 bis 5 mm aufweist.

## Revendications

1. Procédé de préparation d'un produit de comblement de tissu mou, le procédé comprenant les étapes de :
(i) préparation d'un matériau d'hydrogel réticulé qui comprend de l'acide hyaluronique réticulé ou un sel d'acide hyaluronique réticulé pharmaceutiquement acceptable ;
(ii) passage du matériau d'hydrogel réticulé à travers un tamis pour former de multiples filaments d'hydrogel réticulé ; et
(iii) emballage du produit d'hydrogel pour une utilisation comme comblement de tissu mou injectable alors que l'hydrogel réticulé est sous la forme de multiples filaments ;
dans lequel la taille de pore de tamis est de 1 à 200 µm, et chaque filament d'hydrogel réticulé a un diamètre de 1 à 200 µm et une longueur qui est au moins deux fois la taille du diamètre.

2. Procédé selon la revendication 1, dans lequel le matériau d'hydrogel réticulé est passé à travers le tamis une seule fois avant d'être emballé pour une utilisation comme produit de comblement de tissu mou.

3. Procédé selon la revendication 1 ou la revendication 2, dans lequel l'étape de préparation d'un matériau d'hydrogel réticulé comprend la combinaison d'acide hyaluronique ou d'un sel de celui-ci pharmaceutiquement acceptable avec au moins un agent de réticulation choisi parmi le diglycidyléther de 1,4-butanediol (BDDE), le 1,4-bis(2,3-époxypropoxy)butane, le 1,4-bisglycidyloxybutane, le 1,2-bis(2,3-époxypropoxy)éthylène, le 1-(2,3-époxypropyl)-2,3-époxycyclohexane et la divinylsulfone (DVS).

4. Procédé selon l'une quelconque des revendications précédentes, dans lequel le matériau d'hydrogel réticulé comprend du hyaluronate de sodium réticulé.

5. Procédé selon la revendication 4, dans lequel le hyaluronate de sodium est réticulé avec du BDDE.

6. Procédé selon la revendication 1, comprenant en outre l'étape d'addition d'un lubrifiant aux multiples filaments avant l'étape d'emballage.

7. Procédé selon la revendication 6, dans lequel le lubrifiant est un hydrogel non réticulé.

8. Comblement de tissu mou, comprenant un produit d'hydrogel qui comprend de multiples filaments d'hydrogel, les filaments d'hydrogel comprenant de l'acide hyaluronique réticulé ou un sel d'acide hyaluronique réticulé pharmaceutiquement acceptable, et les filaments d'hydrogel ayant un diamètre de 1 à 200 µm et une longueur qui est au moins deux fois la taille du diamètre.

9. Comblement selon la revendication 8, dans lequel chaque filament d'hydrogel a un diamètre de 1 à 60 µm.

10. Comblement selon la revendication 9, dans lequel chaque filament d'hydrogel a un diamètre de 25 à 60 µm.

11. Comblement selon la revendication 8, dans lequel chaque filament d'hydrogel a une longueur d'au moins 0,1 mm.

12. Comblement selon la revendication 11, dans lequel chaque filament d'hydrogel a une longueur de 0,1 à 5 mm.

13. Comblement selon la revendication 12, dans lequel chaque filament d'hydrogel a une longueur de 0,5 à 3 mm.

14. Comblement selon la revendication 8, dans lequel chaque filament d'hydrogel a un diamètre de 25 à 60 µm et une longueur de 0,1 à 5 mm.
